# EUROPÄISCHE PATENTANMELDUNG

(11) **EP 4 563 560 A1**
(43) Veröffentlichungstag der Anmeldung: **04.06.2025**
(21) Anmeldenummer: 24213337.9
(22) Anmeldetag: 15.11.2024
(51) Int. Cl.: C07C 7/00, C07C 7/04, C07C 11/08, B01D 1/28, B01D 3/14

(54) **ENERGIEEFFIZIENTES VERFAHREN ZUR ABTRENNUNG VON 1-BUTEN AUS EINEM KOHLENWASSERSTOFFSTROM MIT OPTIMIERTER BRÜDENVERDICHTUNG**

(30) Priorität: 29.11.2023 EP 23213040
(71) Anmelder: Evonik Oxeno GmbH & Co. KG, 45772 Marl (DE)
(72) Erfinder: Paul, Niklas, 45770 Marl (DE); Rix, Armin Matthias, 45770 Marl (DE); Six, Tanita Valèrie, 44309 Dortmund (DE); Steenweg, Tanja, 44139 Dortmund (DE); Peitz, Stephan, 45739 Oer-Erkenschwick (DE); Stochniol, Guido, 45721 Haltern am See (DE); Fleischer, Vinzenz, 45770 Marl (DE); Wessner, Lea, 44139 Dortmund (DE); Schröder, Moritz, 48153 Münster (DE)
(74) Vertreter: Evonik Patent Association

(57) **Zusammenfassung**

Gegenstand der Erfindung ist ein Verfahren zur Abtrennung von 1-Buten aus einem Kohlenwasserstoffstrom, der zumindest 1-Buten, 2-Buten, n-Butan und Isobutan enthält, in einer Abtrenneinheit, die mindestens zwei Destillationskolonnen DK1 und DK2 umfasst, wobei die Kondensationswärme durch optimierte Brüdenverdichtung in den Prozess rezirkuliert wird, um Energiekosten und CO₂-Emissionen einzusparen.

## Beschreibung

Die vorliegende Erfindung betrifft ein Verfahren zur Abtrennung von 1-Buten aus einem Kohlenwasserstoffstrom, der zumindest 1-Buten, 2-Buten, n-Butan und Isobutan enthält, in einer Abtrenneinheit, die mindestens zwei Destillationskolonnen DK1 und DK2 umfasst, wobei die Kondensationswärme durch optimierte Brüdenverdichtung in den Prozess rezirkuliert wird, um Energiekosten und CO₂-Emissionen einzusparen.

1-Buten kann in großen Mengen aus technischen C4-Kohlenwasserstoffströmen, beispielsweise dem C4-Schnitt aus Steamcrackern oder FCC-Einheiten, gewonnen werden. Diese C4-Kohlenwasserstoffströme bestehen im Wesentlichen aus Butadien, den Monoolefinen Isobuten, 1-Buten und den beiden 2-Butenen (cis- und trans-2-Buten) sowie den gesättigten Kohlenwasserstoffen Isobutan und n-Butan. Wegen der geringen Siedepunktsunterschiede der Inhaltsstoffe, deren geringen Trennfaktoren und der Ausbildung von Azeotropen ist eine ausschließlich destillative Aufarbeitung von C4-Kohlenwasserstoffströmen schwierig und nicht wirtschaftlich.

Üblicherweise wird deshalb zunächst das Butadien mittels Extraktivdestillation abgetrennt oder selektiv zu Butenen hydriert. Zurück bleibt jeweils ein C4-Kohlenwasserstoffstrom (sogenanntes Raffinat 1), der neben den gesättigten Kohlenwasserstoffen n-Butan und Isobutan die Olefine Isobuten, 1-Buten und 2-Butene enthält, während das Butadien maximal in geringen Mengen vorhanden ist.

Da die Siedepunkte von 1-Buten und Isobuten eng beieinanderliegen, kann aus entsprechenden C4-Kohlenwasserstoffströmen in der Regel kein 1-Buten über einfache Destillation wirtschaftlich abgetrennt werden. Das Isobuten wird deshalb möglichst weitgehend entfernt, beispielsweise durch Veretherung mit Alkoholen, z.B. Methanol oder Ethanol zu MTBE- oder ETBE. Durch die weitgehende reaktive Entfernung des Isobutens entsteht ein C4-Kohlenwasserstoffstrom (sogenanntes Raffinat 2), der die linearen Butene (1- und 2-Butene) und die gesättigten Kohlenwasserstoffe Isobutan und n-Butan enthält.

Die Abtrennung von 1-Buten aus derartigen C4-Kohlenwasserstoffströmen ist möglich und wird in der chemischen Industrie eingesetzt. Die Abtrennung erfolgt dabei in einer Destillationseinheit, die mindestens zwei Destillationskolonnen umfasst. In der ersten Destillationskolonne werden Isobutan und 1-Buten am Kopf anfallen und zur zweiten Destillationskolonne geleitet. In der zweiten Destillationskolonne werden dann Isobutan und 1-Buten voneinander getrennt. Ein solches Verfahren wird beispielsweise in der DE 10 2005 062 700 A1 offenbart.

Die für die Auftrennung des C4-Kohlenwasserstoffstroms notwendige Energie wird bei den bekannten Verfahren üblicherweise über Heizdampf in den Sumpf der beiden Destillationskolonnen eingebracht. Zwar steht an chemischen Produktionsstandorten Heizdampf in aller Regel zur Verfügung. In den für die betreffenden Trennaufgaben benötigten Mengen, bedeutet der Einsatz von Heizdampf einen nicht zu unterschätzenden Kostenfaktor. Darüber hinaus wird bei der Erzeugung von Heizdampf eine große Menge an CO₂ erzeugt.

Die Aufgabe der vorliegenden Erfindung bestand deshalb darin ein Verfahren bereitzustellen, bei dem Energie und CO₂-Emissionen im Vergleich zu den bekannten Verfahren eingespart werden können und das in bestehende Anlagen integriert werden kann.

Diese Aufgabe wird durch die in Anspruch 1 vorgeschlagene Ausführung des Verfahrens gelöst. Bevorzugte Ausführungsformen sind in den Unteransprüchen angegeben. Das erfindungsgemäße Verfahren ist ein Verfahren zur Abtrennung von 1-Buten aus einem Raffinat-2-Strom, der zumindest 1-Buten, 2-Buten, n-Butan, Isobuten und Isobutan enthält, in einer Abtrenneinheit, die mindestens zwei Destillationskolonnen DK1 und DK2 umfasst, wobei
die erste Destillationskolonne DK1 mindestens einen Sumpfverdampfer SV1 und die zweite Destillationskolonne DK2 mindestens einen Sumpfverdampfer SV2 aufweist;
der Sumpfverdampfer SV1 mit einem Strom gespeist wird, der am unteren Ende der DK1 entnommen wird und nach Durchlaufen des jeweiligen Sumpfverdampfers wieder zur DK1 geführt wird;
der Sumpfverdampfer SV2 mit einem Strom gespeist wird, der am unteren Ende der DK2 entnommen wird und nach Durchlaufen des jeweiligen Sumpfverdampfers wieder zur DK2 geführt wird; wobei das Verfahren die folgenden Schritte umfasst
   (a) der Raffinat-2-Strom zur ersten Destillationskolonne DK1 geleitet wird und in der DK1 in mindestens einen Brüdenstrom BS1, der zumindest 1-Buten und Isobutan umfasst und am Kopf der DK1 entnommen wird, und in mindestens einen Sumpfstrom, der zumindest 1-Buten, n-Butan und 2-Buten umfasst und am Sumpf der DK1 entnommen wird, aufgetrennt wird;
   (b) zumindest ein Teil des Brüdenstroms BS1 verdichtet wird, wodurch ein gegenüber dem Brüdenstrom BS1 verdichteter Strom VB1 entsteht;
   (c) Energie vom verdichteten Strom VB1 auf den Strom im Sumpfverdampfer SV1 übertragen wird;
   (d) der Strom VB1 zumindest teilweise zur zweiten Destillationskolonne DK2 geleitet werden und in der DK2 in mindestens einen Brüdenstrom BS2, der zumindest Isobutan umfasst und am Kopf der DK2 entnommen wird, und in mindestens einen Sumpfstrom, der zumindest 1-Buten umfasst und am Sumpf der DK2 entnommen wird, aufgetrennt wird;
   (e) der Brüdenstrom BS2 zumindest teilweise verdichtet wird, wodurch ein gegenüber dem Brüdenstrom BS2 verdichteter Strom VB2 entsteht; und
   (f) Energie vom verdichteten Strom VB2 auf den Strom im Sumpfverdampfer SV2 übertragen wird.

Ein Vorteil des erfindungsgemäßen Verfahrens ist, dass die anfallenden Brüdenströme BS1 und BS2 beider Kolonnen DK1 und DK2 einer Verdichtung unterzogen, demzufolge aufgewertet und in den Sumpfverdampfern SV1 und SV2 ausgenutzt werden und so Energie in die jeweiligen Sümpfe eingetragen wird. Die Energieübertragung in den Sumpfverdampfern SV1 und SV2 hat zur Folge, dass bedeutend weniger oder gar kein Heizdampf zur Beheizung der Destillationskolonnen DK1 und DK2 eingesetzt werden muss. Es kann also eine (fast) vollständige Verstromung des energieintensiven Verfahrens erzielt werden, was wiederum den Einsatz von grünem Strom ermöglicht. Dadurch werden Energiekosten und CO₂-Emissionen eingespart.

Der Ausgangsstrom, aus dem das 1-Buten abgetrennt werden soll, ist nach der vorliegenden Erfindung ein Raffinat-2-Strom, der zumindest 1-Buten, 2-Buten, n-Butan und Isobutan enthält. Entsprechende Ströme sind auf dem Markt verfügbar, beispielsweise als C4-Schnitt aus Steamcrackern oder FCC-Einheiten. Es wurde in der Einleitung bereits erwähnt, dass Raffinat 2 dadurch entsteht, dass mehrfach ungesättigte C4-Kohlenwasserstoffe, insbesondere Butadien, und Isobuten aus dem Strom entfernt werden. Eine vollständige Entfernung ist aus wirtschaftlichen und technischen Gründen in vielen Fällen nicht möglich. Die Mengen an mehrfach ungesättigten C4-Kohlenwasserstoffen, insbesondere Butadien, und Isobuten sollten jedoch möglichst gering sein.

Vorzugsweise enthält das eingesetzte Raffinat 2 weniger als 2500 ppm, vorzugsweise weniger als 1000 ppm Isobuten, besonders bevorzugt weniger als 500 ppm Isobuten. Sind höhere Mengen an Isobuten im Ausgangstrom vorhanden, könnte zwischen die beiden Destillationskolonnen DK1 und DK2 eine MTBE- oder ETBE-Synthese (Methyl-tert.-butyl-ether = MTBE / Ethyl-tert.-butyl-ether = ETBE) erfolgen, um das Isobuten mit Methanol (zu MTBE) oder Ethanol (zu ETBE) umzusetzen und anschließend das MTBE oder ETBE abzutrennen (vgl. EP 1 806 330 A1 oder EP1 813 588 A1). So kann die Konzentration an Isobuten vor der zweiten Destillationskolonne DK2 sehr stark reduziert werden. Isobuten würde in der zweiten Destillationskolonne nämlich im Sumpf und damit im 1-Buten anfallen.

Weiterhin bevorzugt enthält das im Verfahren der vorliegenden Erfindung eingesetzte Raffinat 2 weniger als 4 Gew.-% mehrfach ungesättigte C4-Kohlenwasserstoffe. In einer besonders bevorzugten Ausführungsform sollte die Konzentration der mehrfach ungesättigten C4-Kohlenwasserstoffe weniger als 500 ppm betragen. Sollten die Ströme höhere Mengen an Butadien enthalten, könnte vorher eine Selektivhydrierung durchgeführt werden, bei der das Butadien zu Butenen und/oder Butanen umgesetzt wird. Entsprechende Verfahren sind dem Fachmann beispielsweise aus der EP 3 680 224 A1 bekannt.

Der eingesetzte Raffinat-2-Strom kann darüber hinaus gewisse Mengen an Wasser enthalten, insbesondere in einer Menge von 150 bis 4000 ppm. Es ist bevorzugt, dass das Wasser durch das hier beschriebene Verfahren zumindest teilweise abgetrennt wird. Das Wasser wird sich dabei in jeder der beiden Destillationskolonnen DK1 und DK2 im jeweiligen Brüdenstrom BS1 und BS2 anreichern und nach Kondensation als zweite flüssige Phase anfallen, die über Euter in den Destillatbehältern der DK1 und/oder der DK2 abgetrennt werden können. Die Sumpfprodukte der DK1 und der DK2 zeichnen sich durch sehr niedrige Gehalte an Butadien und Wasser, bevorzugt je unter 100 ppm, besonders bevorzugt unter 5 ppm aus.

Das erfindungsgemäße Verfahren wird in einer Abtrenneinheit durchgeführt, die mindestens die beiden Destillationskolonnen DK1 und DK2 umfasst. DK1 ist die erste Destillationskolonne und weist mindestens einen Sumpfverdampfer SV1 auf. DK2 ist die zweite Destillationskolonne und weist mindestens einen Sumpfverdampfer SV2 auf. In einer bevorzugten Ausführungsform weist die Destillationskolonne nur den einen Sumpfverdampfer SV1 auf. Es ist zudem bevorzugt, dass die Destillationskolonne DK2 nur den einen Sumpfverdampfer SV2 aufweist. Die Drücke in den beiden Destillationskolonnen DK1 und DK2 sollten insbesondere so gewählt werden, dass die Trennaufgabe durch die Beeinflussung der relativen Flüchtigkeit erleichtert, aber die Kompression der Volumenströme BS1 und BS2 nicht zu energieintensiv wird. Die Begriffe "erste Destillationskolonne", "Destillationskolonne DK1" und "DK1" sind im Rahmen der vorliegenden Erfindung als synonym zu verstehen. Ebenso sind die sie Begriffe "zweite Destillationskolonne", "Destillationskolonne DK2" und "DK2" im Rahmen der vorliegenden Erfindung als synonym zu verstehen.

Über den Sumpfverdampfer SV1 wird die für die Trennaufgabe notwendige Energie in die erste Destillationskolonne DK1 eingebracht. Der Sumpfverdampfer SV1 wird mit einem Strom gespeist, der am unteren Ende der DK1 entnommen und nach Durchlaufen des jeweiligen Sumpfverdampfers wieder zur DK1 geführt wird. Der Strom wird beim Durchlaufen des Sumpfverdampfers SV1 erhitzt.

Vergleichbares gilt für den Sumpfverdampfer SV2 der zweiten Destillationskolonne DK2, durch den für die Trennaufgabe notwendige Energie eingetragen wird. Der Sumpfverdampfer SV2 wird dazu mit einem Strom gespeist, der am unteren Ende der DK2 entnommen und nach Durchlaufen des jeweiligen Sumpfverdampfers wieder zur DK2 geführt wird. Der Strom wird beim Durchlaufen des Sumpfverdampfers SV2 erhitzt und verdampft dabei zumindest teilweise.

Als "Sumpfverdampfer" werden erfindungsgemäß Verdampfer bezeichnet, die den Sumpf der jeweiligen Destillationskolonne beheizen. Ein derartiger Sumpfverdampfer ist üblicherweise außerhalb der jeweiligen Destillationskolonne angeordnet. Da in Sumpfverdampfern Energie, insbesondere Wärme, von einem Strom auf einen anderen übertragen wird, sind sie Wärmeüberträger. Über einen Abzug aus dem Sumpf der Destillationskolonne wird der zu verdampfende Strom abgezogen und dem Sumpfverdampfer zugeführt. Über mindestens einen Zulauf wird der verdampfte Strom gegebenenfalls mit einem Restanteil Flüssigkeit wieder in die jeweilige Destillationskolonne im Bereich des Sumpfs zurückgeführt.

Geeignete Verdampfer, die als Sumpfverdampfer eingesetzt werden können, sind zum Beispiel Naturumlaufverdampfer, Zwangsumlaufverdampfer, Zwangsumlaufverdampfer mit Entspannung, Kesselverdampfer, Fallfilmverdampfer oder Dünnschichtverdampfer. Als Wärmeübertrager für den Verdampfer wird bei Naturumlaufverdampfern und Zwangsumlaufverdampfern üblicherweise ein Rohrbündel oder Plattenapparat eingesetzt. Neben den genannten kann aber auch jede beliebige andere, dem Fachmann bekannte Verdampferbauart, die sich zum Einsatz an einer Destillationskolonne eignet, eingesetzt werden.

Das Raffinat 2, das zur ersten Destillationskolonne DK1 geleitet wird, wird in der Destillationskolonne DK1 in mindestens zwei Ströme aufgetrennt, d. h. in mindestens einen Brüdenstrom BS1, der zumindest 1-Buten und Isobutan umfasst und am Kopf der DK1 entnommen wird, und in mindestens einen Sumpfstrom, der zumindest 1-Buten und 2-Buten umfasst und am Sumpf der DK1 entnommen wird. Dieser Sumpfstrom kann zu einer Oligomerisierung geführt werden (nicht gezeigt). Der Brüdenstrom BS1 kann am Kopf der Destillationskolonne auch in Form von mehreren Teilströmen BS1n, wobei n eine ganze Zahl und gleich der Anzahl der Teilströme ist, entnommen werden. Gleiches gilt auch für den Sumpfstrom. Im Sumpf der ersten Destillationskolonne DK1 liegt vorzugsweise eine Temperatur im Bereich von 40 bis 110 °C, vorzugsweise 50 bis 100 °C vor.

Grundsätzlich kann der Raffinat-2-Strom über eine oder mehrere Zulaufstellen in die erste Destillationskolonne DK1 geleitet werden. Sind starke Schwankungen der Zulaufkonzentration zu erwarten, so kann es sinnvoll sein, mehrere unterschiedliche Feedzulaufstellen vorzusehen. Deren optimale Lage kann z.B. mittels Simulation durch Minimierung des Energiebedarfs gefunden werden. Sollen mehrere Zulaufströme unterschiedlicher Zusammensetzung verarbeitet werden, so kann die optimale Zulaufstelle analog für jeden Feed ermittelt werden. Es ist grundsätzlich auch möglich, dass der Raffinat-2-Strom auch bei gleicher oder gleichbleibender Zusammensetzung vorher in mehrere Teilströme geteilt wird. In diesem Fall wird der Raffinat-2-Strom in Form von zwei oder mehr voneinander getrennten Strömen in die Destillationskolonne DK1 geleitet. Dabei ist es vorteilhaft, wenn die Zulaufstellen der einzelnen Ströme im Wesentlichen auf der gleichen Höhe an der Destillationskolonne DK1 liegen.

Im Folgenden werden der Druck und die Temperatur des Brüdenstroms BS1 angegeben. Dies bezieht sich insbesondere auf den Druck und die Temperatur des mindestens einen Brüdenstroms BS1, wenn er der Destillationskolonne DK1 entnommen wird. Der Druck des Brüdenstroms BS1 liegt insbesondere im Bereich von 6 bis 15 bar absolut, vorzugsweise im Bereich von 7,5 bis 13 bar absolut. Die Temperatur des Brüdenstroms BS1 liegt insbesondere im Bereich von 45 °C bis 120 °C, bevorzugt im Bereich von 48 °C bis 100 °C, weiterhin bevorzugt im Bereich von 50 °C bis 90 °C, weiterhin bevorzugt im Bereich von 55 °C bis 80 °C, besonders bevorzugt im Bereich von 60 °C bis 80 °C.

Als Destillationskolonne DK1 für die Auftrennung des Raffinat-2-Stroms kann jede beliebige, dem Fachmann bekannte Destillationskolonne eingesetzt werden. Bevorzugt enthält die Destillationskolonne DK1 Einbauten. Geeignete Einbauten sind zum Beispiel Böden, unstrukturierte Packungen (Füllkörper) oder strukturierte Packungen. Als Böden werden üblicherweise Glockenböden, Siebböden, Ventilböden mit festen oder beweglichen Ventilen, Tunnelböden oder Schlitzböden eingesetzt. Unstrukturierte Packungen sind im Allgemeinen Füllkörperschüttungen. Als Füllkörper werden üblicherweise Raschigringe, Pallringe, Berl-Sättel, SuperRinge/SuperRinge Plus oder Intalox^{®}-Sättel verwendet. Strukturierte Packungen werden zum Beispiel unter dem Handelsnamen Mellapak^{®} der Firma Sulzer vertrieben. Neben den genannten Einbauten sind dem Fachmann weitere geeignete Einbauten bekannt und können ebenfalls verwendet werden.

Bevorzugte Einbauten weisen einen geringen spezifischen Druckverlust pro theoretischer Trennstufe auf. Strukturierte Packungen und Füllkörper haben zum Beispiel einen deutlich kleineren Druckverlust pro theoretischer Trennstufe als Böden. Dies hat den Vorteil, dass der Druckverlust in der Destillationskolonne DK1 möglichst gering bleibt und somit die mechanische Leistung des Verdichters und die Temperatur des zu verdampfenden Raffinat-2-Stroms gering bleibt.

In einer besonders bevorzugten Ausführungsform der vorliegenden Erfindung umfasst die Destillationskolonne DK1 eine Vielzahl von Böden, vorzugsweise zwischen 150 und 300 Böden, weiterhin bevorzugt zwischen 170 und 220 Böden.

Die Entnahme von mindestens einem Brüdenstroms BS1, der zumindest 1-Buten und Isobutan umfasst, am Kopf der Destillationskolonne DK1 bedeutet im Rahmen der vorliegenden Erfindung insbesondere, dass der mindestens eine Brüdenstrom BS1 als Kopfstrom oder als Seitenabzug oberhalb der Einbauten in der Destillationskolonne DK1 entnommen wird.

Die Entnahme des mindestens einen Sumpfstroms, der zumindest 1-Buten und 2-Buten umfasst, am Sumpf der Destillationskolonne DK1 bedeutet im Rahmen der vorliegenden Erfindung insbesondere, dass der mindestens eine Sumpfstrom direkt am Sumpf oder am unteren Boden der Destillationskolonne DK1 entnommen wird.

Die Destillationskolonne DK1 wird vorzugsweise mit Rücklauf betrieben. Rücklauf bedeutet, dass der am oberen Ende der Destillationskolonne DK1 entnommene Brüdenstrom BS1 mindestens teilweise wieder der Destillationskolonne DK1 zugeführt wird. In den Fällen, in denen ein solcher Rücklauf eingestellt wird, beträgt das Rücklaufverhältnis dabei bevorzugt 2 bis 30, bevorzugter 5 bis 20, besonders bevorzugt 8 bis 15.

Ein Rücklauf kann dadurch eingestellt werden, dass am Kopf der Destillationskolonne DK1 ein Kondensator angebracht wird. In dem Kondensator wird der Brüdenstrom BS1 teilweise kondensiert und der Destillationskolonne DK1 wieder zugeführt. Der Brüdenstrom kann auch erst nach Verdichtung und Entspannung als Rücklauf auf die Destillationskolonne gegeben werden. Unter einem Rücklaufverhältnis wird allgemein und im Sinne dieser Erfindung das Verhältnis aus dem Anteil des aus der Kolonne entnommenen Massenstroms (kg/h), der wieder auf die Kolonne in flüssiger Form zurückgeführt wird (Rücklauf) zu dem Anteil dieses Massenstroms (kg/h), der in flüssiger Form oder gasförmiger Form von der jeweiligen Kolonne abgeführt wird, verstanden.

Nach der Entnahme des Brüdenstroms BS1 wird der Brüdenstroms BS1 verdichtet, wodurch ein gegenüber dem Brüdenstrom BS1 verdichteter Strom VB1 entsteht. Es kann vorteilhaft sein den Strom BS1 vor dem Verdichten zu erwärmen, damit bei der Verdichtung kein zweiphasiges Gemisch entsteht.

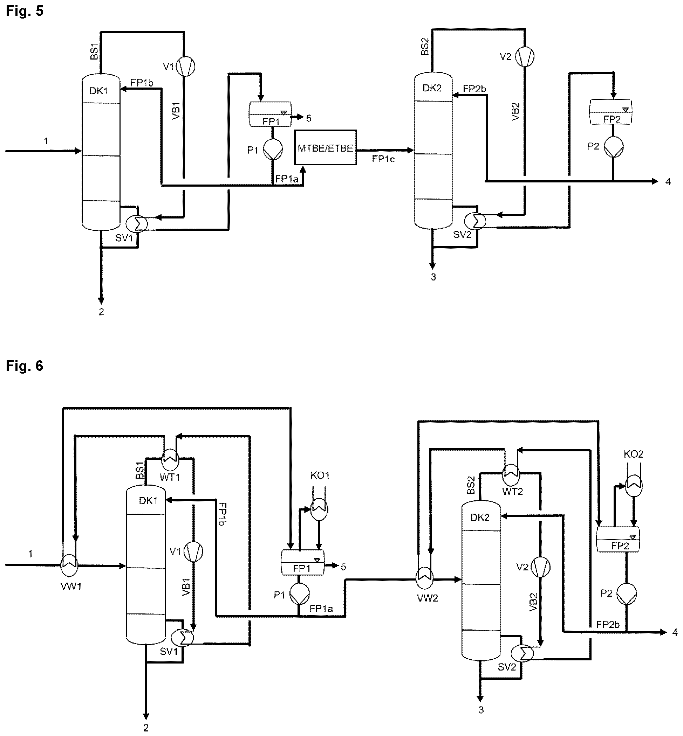

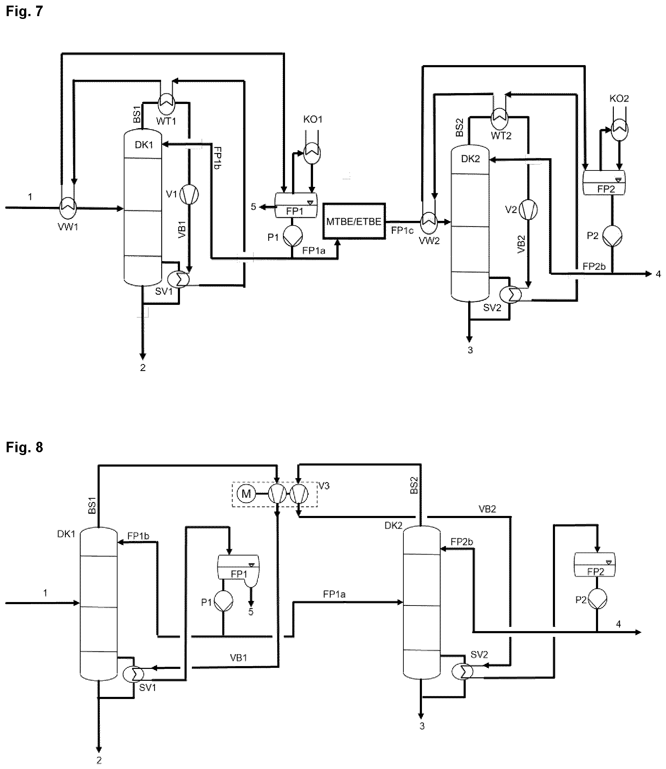

Die Erwärmung kann mittels interner (s. WT1 in Fig. 6 und 7 der vorliegenden Anmeldung) oder externer Wärmequellen durchgeführt werden. Der Druck von VB1 ist nach dem Verdichten höher als der Druck von BS1. Der genaue Wert für den Druck von VB1 kann in Abhängigkeit der Anforderungen bei der nachfolgenden Energieübertragung vom Fachmann eingestellt werden, solange die Bedingung Druck VB1 > Druck BS1 erfüllt ist. Der Quotient aus Druck VB1 / Druck BS1 (Drücke jeweils in bar abs.) liegt bevorzugt im Bereich von 1,1 bis 10, bevorzugter 1,2 bis 8, bevorzugter 1,25 bis 7, am bevorzugtesten 1,3 bis 6.

Die Temperatur des Teilstroms VB1 ist vorzugsweise höher als die Temperatur des Brüdenstroms BS1, und der Quotient aus Temperatur VB1 / Temperatur BS1 (Temperatur jeweils in K) liegt bevorzugt im Bereich von 1,03 bis 10, bevorzugter 1,04 bis 9, bevorzugter 1,05 bis 8, bevorzugter 1,06 bis 7, bevorzugter 1,07 bis 6, am bevorzugtesten 1,08 bis 5.

Das Verdichten zumindest eines Teils des Brüdenstroms BS1 in Schritt (b) kann auf jede beliebige, dem Fachmann bekannte Art erfolgen. So kann die Verdichtung zum Beispiel mechanisch und einstufig oder mehrstufig durchgeführt werden. Einstufig bedeutet in diesem Zusammenhang, dass eine Verdichtung von einem auf ein anderes Druckniveau stattfindet. Mehrstufig bedeutet, dass erst auf ein Druckniveau X und dann von X auf das Druckniveau Y verdichtet wird. Bei einer mehrstufigen Verdichtung können mehrere Verdichter gleicher Bauart oder Verdichter unterschiedlicher Bauart eingesetzt werden. Eine mehrstufige Verdichtung kann bevorzugt mit einer Verdichtermaschine oder mit mehreren Verdichtermaschinen erfolgen. Der Einsatz einer einstufigen Verdichtung oder einer mehrstufigen Verdichtung ist vom Verdichtungsverhältnis und somit davon abhängig, auf welchen Druck der Brüdenstrom BS1 verdichtet werden soll.

Als Verdichter im erfindungsgemäßen Verfahren, insbesondere zum Verdichten des Brüdenstroms BS1 zu VB1, eignet sich jeder beliebige, dem Fachmann bekannte Verdichter, bevorzugt mechanische Verdichter, mit dem sich Gasströme verdichten lassen. Geeignete Verdichter sind zum Beispiel ein- oder mehrstufige Getriebeturboverdichter, Kolbenverdichter, Schraubenverdichter, Zentrifugalverdichter oder Axialverdichter.

Im Schritt (c) des erfindungsgemäßen Verfahrens wird Energie vom verdichteten Strom VB1 auf den Strom im Sumpfverdampfer SV1 übertragen. Durch den Schritt (c) sinkt die Energie von VB1, so dass VB1 teilweise kondensieren kann. Die Phrase "Übertragung von Energie" bedeutet erfindungsgemäß insbesondere Beheizung, also Übertragung von Energie in Form von Wärme.

Die Übertragung von Energie von VB1 auf den Strom im Sumpfverdampfer SV1, bevorzugt die Beheizung des Stroms im Sumpfverdampfer SV1 durch VB1 erfolgt bevorzugt direkt. Direkte Übertragung bedeutet, dass VB1 und der Strom in SV1 sich nicht direkt kontaktieren, aber dass Energie, insbesondere Wärme, von VB1 auf den Strom im SV1 übergeht, ohne dass ein zusätzliches Wärmeüberträgermedium vorhanden ist. Als Sumpfverdampfer SV1 können die dem Fachmann geläufigen Wärmeüberträger bzw. Wärmetauscher, insbesondere Verdampfer, eingesetzt werden.

Bevor der Strom VB1 zur zweiten Destillationskolonne mit Schritt (d) zur zweiten Destillationskolonne geführt werden, wird der Strom VB1 nach einer bevorzugten Ausführungsform zum einem Flashbehälter geführt und dort entspannt, wodurch eine flüssige Phase FP1 anfällt. Der Flashbehälter kann zusätzlich einen Kondensator umfassen, um einen Teil der anfallenden gasförmigen Phase zu kondensieren.

Zumindest ein Teil FP1a der flüssigen Phase FP1 wird anschließend gemäß Schritt (d) zur Destillationskolonne DK2 geleitet. Dazu wird in einer besonders bevorzugten Ausführungsform eine Pumpe eingesetzt. Hier können dem Fachmann bekannte Pumpen eingesetzt werden. Geeignete Pumpen sind beispielsweise Chemie-Normpumpen.

Es ist weiterhin bevorzugt, dass ein von FP1a verschiedener Teil FP1b der flüssigen Phase FP1 als Rücklauf zur ersten Destillationskolonne DK1 zurückgeführt wird. Besonders bevorzugt ist es, dass dabei Energie vom Strom FP1b auf den Raffinat-2-Strom übertragen wird, bevor der Raffinat-2-Strom in die erste Destillationskolonne DK1 eingeleitet wird. Hierdurch wird der Raffinat-2-Strom vorgewärmt. Dies ist energetisch vorteilhaft, weil weniger Energie für die Trennaufgabe über die Sumpfverdampfer eingebracht werden muss. Die Übertragung von Energie von FP1b auf den Raffinat-2-Strom, bevorzugt die Beheizung des Raffinat-2-Stroms durch FP1b erfolgt bevorzugt direkt, d. h. ohne Einsatz eines (weiteren) Wärmeträgers. Dafür können dem Fachmann geläufige Wärmeüberträger bzw. Wärmetauscher eingesetzt werden.

Die Ströme VB1 bzw. FP1a werden also zur zweiten Destillationskolonne DK2 geführt. Sind die Mengen an Isobuten im Strom VB1 bzw. im Strom FP1a zu hoch, um die Spezifikation des 1-Buten-Produkts der DK2 zu erfüllen, kann, wie erwähnt, eine zusätzliche MTBE oder ETBE-Synthese zwischen den Destillationskolonnen DK1 und DK2 angeordnet sein. Dafür wird der Strom VB1 oder der Strom FP1a zu einer MTBE oder ETBE-Synthese geführt und das enthaltenen Isobuten zumindest teilweise zu MTBE oder ETBE umgesetzt.

Die MTBE- oder ETBE-Synthese ist dem Fachmann grundsätzlich bekannt. Für die Herstellung von MTBE oder ETBE aus isobutenhaltigen Strömen können insbesondere saure lonenaustauschharze (Sulfonsäuregruppen) als heterogene Katalysatoren eingesetzt. Die MTBE- oder ETBE-Synthese kann in einem oder mehreren in Reihe geschalteten Reaktoren stattfinden. Der Katalysator wird vorzugsweise als Festbettkatalysator eingesetzt. Da die Bildung von MTBE oder ETBE eine Gleichgewichtsreaktion darstellt, kann es zweckmäßig sein mindestens eine Reaktivdestillationskolonne einzusetzen, in der gleichzeitig die Reaktion und die Abtrennung des MTBE bzw. des ETBE erfolgen. Bei der Reaktivdestillation sollte ein Druck im Bereich von 3 bis 15 bar und eine Temperatur in der Reaktionszone von 55 bis 75 °C vorliegen.

Nach der Synthese werden MTBE oder ETBE vom Strom VB1 oder FP1a abgetrennt, vorzugsweise mittels Destillation. Auch dieses Verfahren ist dem Fachmann bekannt. Erst dann werden VB1 oder FP1a zur Destillationskolonne DK2 geführt.

In der zweiten Destillationskolonne DK2 werden die Ströme, die beide jeweils zumindest Isobutan und 1-Buten umfassen, gemäß Schritt (d) in mindestens einen Brüdenstrom BS2, der zumindest Isobutan umfasst und am Kopf der DK2 entnommen wird, und in mindestens einen Produktstrom, der zumindest 1-Buten umfasst und am Sumpf der DK2 entnommen wird, aufgetrennt.

Als Destillationskolonne DK2 für die Auftrennung des Raffinat-2-Stroms kann jede beliebige, dem Fachmann bekannte Destillationskolonne eingesetzt werden. Bevorzugt enthält die Destillationskolonne DK2 Einbauten. Geeignete Einbauten sind zum Beispiel Böden, unstrukturierte Packungen (Füllkörper) oder strukturierte Packungen. Als Böden werden üblicherweise Glockenböden, Siebböden, Ventilböden mit festen oder beweglichen Ventilen, Tunnelböden oder Schlitzböden eingesetzt. Unstrukturierte Packungen sind im Allgemeinen Füllkörperschüttungen. Als Füllkörper werden üblicherweise Raschigringe, Pallringe, Berl-Sättel, SuperRinge/SuperRinge Plus oder Intalox^{®}-Sättel verwendet. Strukturierte Packungen werden zum Beispiel unter dem Handelsnamen Mellapak^{®} der Firma Sulzer vertrieben. Neben den genannten Einbauten sind dem Fachmann weitere geeignete Einbauten bekannt und können ebenfalls verwendet werden.

Bevorzugte Einbauten weisen einen geringen spezifischen Druckverlust pro theoretischer Trennstufe auf. Strukturierte Packungen und Füllkörper haben zum Beispiel einen deutlich kleineren Druckverlust pro theoretischer Trennstufe als Böden. Dies hat den Vorteil, dass der Druckverlust in der Destillationskolonne DK2 möglichst gering bleibt und somit die mechanische Leistung des Verdichters und die Temperatur des zu verdampfenden Stroms gering bleibt.

In einer besonders bevorzugten Ausführungsform der vorliegenden Erfindung umfasst die zweite Destillationskolonne DK2 eine Vielzahl von Böden, vorzugsweise zwischen 150 und 300 Böden, weiterhin bevorzugt zwischen 170 und 220 Böden.

Die Entnahme mindestens einen Brüdenstroms BS2, der zumindest Isobutan umfasst, am Kopf der Destillationskolonne DK2 bedeutet im Rahmen der vorliegenden Erfindung insbesondere, dass der mindestens eine Brüdenstroms BS2 als Kopfstrom oder als Seitenabzug oberhalb der Einbauten in der Destillationskolonne DK2 entnommen wird.

Die Entnahme des mindestens einen Produktstroms, der zumindest 1-Buten umfasst, am Sumpf der Destillationskolonne DK2 bedeutet im Rahmen der vorliegenden Erfindung insbesondere, dass der mindestens eine Produktstrom direkt am Sumpf oder am unteren Boden der Destillationskolonne DK2 entnommen wird. Der Produktstrom enthält vorzugsweise mindestens 99 Gew.-% 1-Buten, weiterhin bevorzugt mindestens 99,5 Gew.-% 1-Buten, besonders bevorzugt mindestens 99,6 Gew.-% 1-Buten. Das 1-Buten ist das Zielprodukt des vorliegenden Verfahrens, weshalb der Produktstrom aus dem Verfahren ausgeschleust wird. Das 1-Buten kann beispielsweise als Co-Monomer bei der Herstellung von Polyethylen eingesetzt werden.

Hierbei gilt zu beachten, dass die Trennschärfe in der ersten Destillationskolonne DK1 letztlich die Reinheit vom 1-Buten im Produktstrom, der aus der zweiten Destillationskolonne DK2 entnommen wird, bestimmt. N-Butan und die 2-Butene werden nämlich in der DK2 ebenfalls als Schwersieder und deshalb mit dem 1-Buten anfallen. Dadurch würde das 1-Buten verunreinigt. Es sollte also darauf geachtet werden, dass die Auftrennung des Raffinat-2-Stroms in der DK1 möglichst so gefahren wird, dass kaum n-Butan zur DK2 gelangt. Deshalb ist es bevorzugt, dass der zur DK2 geführte Strom (1a, VB1 bzw. FP1a) unter 1500 ppm, bevorzugt unter 1200 ppm, besonders bevorzugt unter 900 ppm n-Butan enthält, bezogen auf die Gesamtmenge des Stroms.

Im Sumpf der zweiten Destillationskolonne DK2 liegt während des erfindungsgemäßen Verfahrens vorzugsweise eine Temperatur im Bereich von 30 bis 100 °C, vorzugsweise 45 bis 80 °C vor. Weiterhin bevorzugt liegt am Kopf der zweiten Destillationskolonne DK2 ein Druck im Bereich von 3 bis 12 bar absolut, vorzugsweise 5 bis 10 bar absolut vor.

Die Destillationskolonne DK2 kann weiterhin mit Rücklauf betrieben werden. Rücklauf bedeutet, dass der am oberen Ende der Destillationskolonne DK2 entnommene Brüdenstrom BS2 mindestens teilweise wieder der Destillationskolonne DK2 zugeführt wird. In den Fällen, in denen ein solcher Rücklauf eingestellt wird, beträgt das Rücklaufverhältnis dabei bevorzugt 10 bis 100, besonders bevorzugt 30 bis 50.

Die Druckbereiche für den Kopfdruck, also den Druck am Kopf der Destillationskolonnen DK1 und DK2, wurden in den vorherigen Abschnitten definiert. Grundsätzlich wäre es demzufolge möglich, dass die beiden Kolonnen in einer Zweidruckschaltung, also bei einem unterschiedlichen Druck betrieben werden. Von der Hochdruckkolonne kann dann Energie auf die Niederdruckkolonne übertragen werden, zusätzlich zu der hier beschriebenen Brüdenverdichtung. Es ist erfindungsgemäß jedoch bevorzugt, dass die Destillationskolonnen DK1 und DK2 bei gleichem Kopfdruck oder einem ähnlichen Kopfdruck betrieben werden, wobei sich die Kopfdrücke im Falle ähnlicher Kopfdrücke um maximal 20%, vorzugsweise maximal 15% unterscheiden. Vorteilhaft ist neben einem geringeren apparativen Aufwand auch ein geringerer Einsatz von Energie. Schließlich wird die relative Flüchtigkeit mit steigendem Druck sinken und die benötigte Menge an Energie für die zu bewältigende Trennaufgabe in mindestens der Hochdruckkolonne deutlich größer.

Anschließend wird in Schritt (e) ein der Brüdenstroms BS2 zumindest teilweise verdichtet, wodurch ein gegenüber dem Brüdenstrom BS2 verdichteter Strom VB2 entsteht. Der Druck von VB2 ist nach dem Verdichten höher als der Druck von BS2. Der genaue Wert für den Druck von VB2 kann in Abhängigkeit der Anforderungen bei der nachfolgenden Energieübertragung vom Fachmann eingestellt werden, solange die Bedingung Druck VB2 > Druck BS2 erfüllt ist. Der Quotient aus Druck VB2 / Druck BS2 (Drücke jeweils in bar abs.) liegt bevorzugt im Bereich von 1,1 bis 10, bevorzugter 1,2 bis 8, bevorzugter 1,25 bis 7, am bevorzugtesten 1,3 bis 6. Es kann vorteilhaft sein den Strom BS2 vor dem Verdichten zu erwärmen, damit bei der Verdichtung kein zweiphasiges Gemisch entsteht. Die Erwärmung kann mittels interner (s. WT-2 in Fig. 6 und 7 der vorliegenden Anmeldung) oder externer Wärmequellen durchgeführt werden.

Die Temperatur des Teilstroms VB2 ist insbesondere höher als die Temperatur des Brüdenstroms BS2, und der Quotient aus Temperatur VB2 / Temperatur BS2 (Temperatur jeweils in K) liegt bevorzugt im Bereich von 1,03 bis 10, bevorzugter 1,04 bis 9, bevorzugter 1,05 bis 8, bevorzugter 1,06 bis 7, bevorzugter 1,07 bis 6, am bevorzugtesten 1,08 bis 5.

Das Verdichten des mindestens einen Teils des Brüdenstroms BS2 in Schritt (e) kann auf jede beliebige, dem Fachmann bekannte Art erfolgen. So kann die Verdichtung zum Beispiel mechanisch und einstufig oder mehrstufig durchgeführt werden. Bei einer mehrstufigen Verdichtung können mehrere Verdichter gleicher Bauart oder Verdichter unterschiedlicher Bauart eingesetzt werden. Eine mehrstufige Verdichtung kann mit einer oder mehreren Verdichtermaschinen erfolgen. Der Einsatz einer einstufigen Verdichtung oder einer mehrstufigen Verdichtung ist abhängig vom Verdichtungsverhältnis und somit, auf welchen Druck der Brüdenstrom BS2 verdichtet werden soll.

Als Verdichter im erfindungsgemäßen Verfahren, insbesondere zum Verdichten des Brüdenstroms BS2 zu VB2, eignet sich jeder beliebige, dem Fachmann bekannte Verdichter, bevorzugt mechanische Verdichter, mit dem sich Gasströme verdichten lassen. Geeignete Verdichter sind zum Beispiel ein- oder mehrstufige Turbinen, Kolbenverdichter, Schraubenverdichter, Zentrifugalverdichter oder Axialverdichter.

Im Schritt (f) des erfindungsgemäßen Verfahrens wird Energie vom verdichteten Strom VB2 auf den Strom im Sumpfverdampfer SV2 übertragen. Durch den Schritt (f) sinkt die Energie von VB2, so dass VB2 mindestens teilweise kondensieren kann. Die Phrase "Übertragung von Energie" bedeutet erfindungsgemäß insbesondere Beheizung, also Übertragung von Energie in Form von Wärme.

Die Übertragung von Energie von VB2 auf den Strom im Sumpfverdampfer SV2, bevorzugt die Beheizung des Stroms im Sumpfverdampfer SV2 durch VB2 erfolgt bevorzugt direkt. Direkte Übertragung bedeutet, dass VB2 und der Strom in SV2 sich nicht kontaktieren, aber dass Energie, insbesondere Wärme, von VB2 auf den Strom im SV2 übergeht, ohne dass ein zusätzliches Wärmeüberträgermedium vorhanden ist. Als Sumpfverdampfer SV2 können die dem Fachmann geläufigen Wärmeüberträger bzw. Wärmetauscher, insbesondere Verdampfer, eingesetzt werden.

Nachdem der Strom VB2 den Sumpfverdampfer SV2 durchlaufen und Energie auf den dort vorhandenen Strom übertragen hat, kann VB2 als Isobutan-Strom IB1 aus dem Verfahren ausgeschleust werden. Bevor VB2 aber als IB1 aus dem Verfahren abgeführt wird, wird der Strom VB2 nach einer bevorzugten Ausführungsform der vorliegenden Erfindung zum einem Flashbehälter geführt und dort entspannt, wodurch eine flüssige Phase FP2 anfällt. Der Flashbehälter kann zusätzlich einen Kondensator umfassen, um einen Teil der anfallenden gasförmigen Phase zu kondensieren.

Zumindest ein Teil FP2a der flüssigen Phase FP2 kann anschließend als Isobutan-Strom IB1 aus dem Verfahren ausgeschleust werden. Dazu wird in einer besonders bevorzugten Ausführungsform eine Pumpe eingesetzt. Unter Umständen und bei ausreichendem Druckverhältnis wäre es auch möglich, dass keine Pumpe eingesetzt wird. Sofern eine Pumpe eingesetzt wird, können dem Fachmann bekannte Pumpen eingesetzt werden. Geeignete Pumpen sind beispielsweise Chemie-Normpumpen. Es ist weiterhin bevorzugt, dass ein von FP2a verschiedener Teil FP2b der flüssigen Phase FP1 als Rücklauf zur ersten Destillationskolonne DK2 zurückgeführt wird.

In der grundsätzlichen Ausgestaltung der vorliegenden Erfindung werden die beiden Ströme BS1 und BS2 jeweils mit einem einzigen Verdichter verdichtet. In einer bevorzugten Ausführungsform der vorliegenden Erfindung werden die beiden Ströme BS1 und BS2 in einer einzigen Verdichtermaschine, vorzugsweise mehrstufig verdichtet. Die Anzahl der notwendigen Stufen hängt davon ab, welches Verdichtungsverhältnis für die jeweiligen Brüdenströme BS1 und BS2 angestrebt werden.

Die Wärmeintegration durch die hier beschriebene Brüdenverdichtung ließe sich auch mit anderen Maßnahmen zur Wärmeintegration kombinieren. Möglich wäre es hier noch eine oder mehrere Wärmepumpe(n) vorzusehen.

Die vorliegende Erfindung wird nachfolgend anhand von Abbildungen erläutert. Die Abbildungen dienen der Veranschaulichung, sind aber nicht einschränkend zu verstehen.

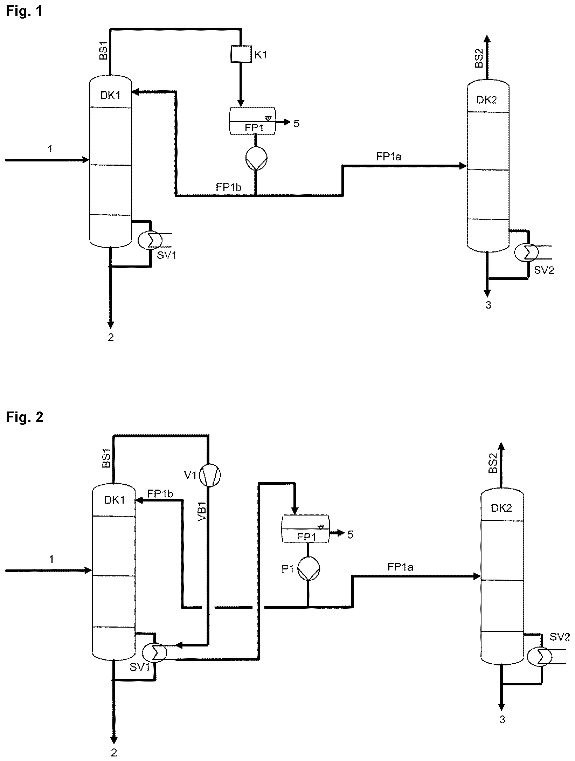

Fig. 1 zeigt eine Ausführungsform nach dem Stand der Technik ohne die erfindungsgemäße Wärmeintegration. Der Raffinat-2-Strom (1) wird zur ersten Destillationskolonne DK1 geführt und dort in einen Brüdenstrom BS1, der zumindest 1-Buten und Isobutan umfasst und am Kopf der DK1 entnommen wird, und in einen Sumpfstrom (2), der zumindest 1-Buten und 2-Buten umfasst und am Sumpf der DK1 entnommen wird. Dieser Strom (2) kann zu einer Oligomerisierung (nicht eingezeichnet) geführt werden. Der Energieeintrag in die Destillationskolonne DK1 erfolgt, indem Energie auf den Strom im Sumpfverdampfer SV1 übertragen wird. Der Brüdenstrom BS1 wird über eine Kühlvorrichtung (K1), beispielsweise einen Luftkühler oder einen mit Kühlwasser betriebenen Wärmeübertrager, zumindest teilweise kondensiert und zu einem Vorlagebehälter gefahren, wo die flüssige Phase FP1 und FP1w anfallen können. Diese organische flüssige Phase (FP1) wird in die beiden Ströme FP1a, der zur zweiten Destillationskolonne DK2 geführt wird, und FP1b, der als Rücklauf zur ersten Destillationskolonne geleitet wird, aufgetrennt. Außerdem kann eine schwere wässrige Phase (5) abgetrennt werden. In der Destillationskolonne DK2 erfolgt die Auftrennung in einen Brüdenstrom BS2, der zumindest Isobutan umfasst und am Kopf der DK2 entnommen wird, und in einen Produktstrom (3), der zumindest 1-Buten umfasst und am Sumpf der DK2 entnommen wird. Der Energieeintrag in die Destillationskolonne DK2 erfolgt, indem Energie auf den Strom im Sumpfverdampfer SV2 übertragen wird. Die Übertragung erfolgt mit Heizdampf.

Fig. 2 zeigt ebenfalls eine nicht erfindungsgemäße Ausführungsform, bei der nur an der Destillationskolonne DK1 eine Wärme-Rückgewinnung stattfindet. Im Unterschied zu der in Fig. 1 gezeigten Ausführungsform, wird vorliegend der Brüdenstrom BS1 mit einem Verdichter V1 auf einen höheren Druck gebracht und anschließend als Strom VB1 zum Sumpfverdampfer SV1 geleitet. Im Sumpfverdampfer SV1 wird Energie auf den dort vorhandenen Strom, der aus dem Sumpf kommend erhitzt und dann wieder zurückgeführt wird, übertragen, um die für die Auftrennung notwendige Energie einzutragen. VB1 wird anschließend zum Flashbehälter geführt, wo die flüssige Phase FP1 anfällt, die als Rücklauf FP1b zurück auf die oberste Trennstufe der Kolonne und als FP1a als Feed zur DK2 gefahren wird. Außerdem kann eine schwere wässrige Phase (5) abgetrennt werden.

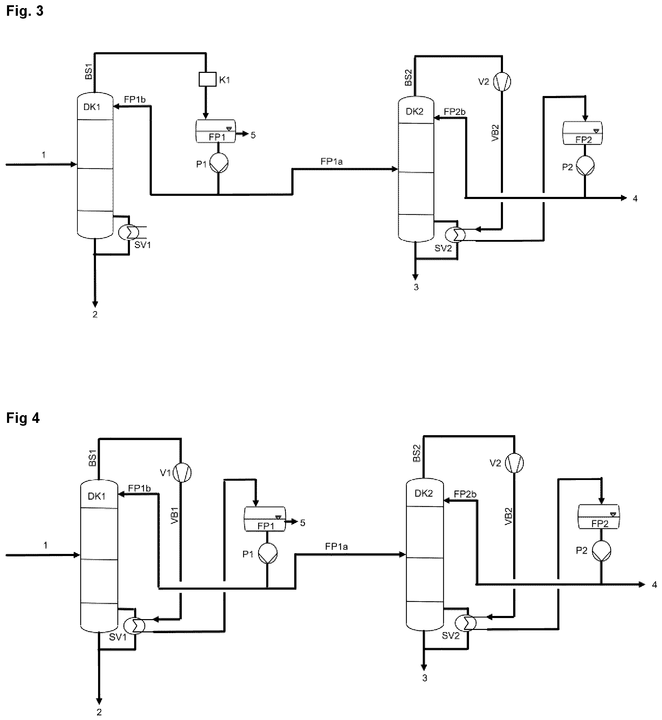

Fig. 3 zeigt ebenfalls eine nicht erfindungsgemäße Ausführungsform, bei der nur an der Destillationskolonne DK2 eine Wärmeintegration stattfindet. Im Unterschied zu der in Fig. 1 gezeigten Ausführungsform, wird vorliegend der Brüdenstrom BS2 mit einem Verdichter V2 auf einen höheren Druck gebracht und anschließend als Strom VB2 zum Sumpfverdampfer SV2 geleitet. Im Sumpfverdampfer SV2 wird Energie auf den dort vorhandenen Strom, der aus dem Sumpf kommend erhitzt und dann wieder zurückgeführt wird, übertragen, um die für die Auftrennung notwendige Energie einzutragen. Der Strom VB2 wird zu einem Flashbehälter gefahren, wo durch die Entspannung eine flüssige Phase FP2 anfällt. Diese flüssige Phase (FP2) wird in die beiden Ströme FP2b, der als Rücklauf zur zweiten Destillationskolonne DK2 zurückgeführt wird, und einen Isobutan-Strom (4), der aus dem Verfahren ausgeschleust wird, aufgetrennt. Außerdem kann eine schwere wässrige Phase (5) abgetrennt werden.

Fig. 4 zeigt die grundsätzliche Ausgestaltung der vorliegenden Erfindung. Der Raffinat-2-Strom (1) wird zur ersten Destillationskolonne DK1 geführt und dort in einen Brüdenstrom BS1, der zumindest 1-Buten und Isobutan umfasst und am Kopf der DK1 entnommen wird, und in einen Sumpfstrom (2), der zumindest 1-Buten und 2-Buten umfasst und am Sumpf der DK1 entnommen wird. Dieser Strom (2) kann zu einer Oligomerisierung (nicht eingezeichnet) geführt werden. Der Brüdenstrom BS1 wird mit dem Verdichter V1 auf einen höheren Druck gebracht und anschließend als Strom VB1 zum Sumpfverdampfer SV1 geführt und überträgt dort Energie auf den dort vorhandenen Strom, der aus dem Sumpf kommend erhitzt und dann wieder zurückgeführt wird. Der Strom VB1 wird anschließend zu einem Flashbehälter gefahren, wo durch die Entspannung eine flüssige Phase FP1 anfällt. Diese flüssige Phase (FP1) wird in die beiden Ströme FP1a, der zur zweiten Destillationskolonne DK2 geführt wird, und FP1b, der als Rücklauf zur ersten Destillationskolonne DK1 zurückgeführt wird, aufgetrennt. In der Destillationskolonne DK2 erfolgt die Auftrennung in einen Brüdenstrom BS2, der zumindest Isobutan umfasst und am Kopf der DK2 entnommen wird, und in einen Produktstrom (3), der zumindest 1-Buten umfasst und am Sumpf der DK2 entnommen wird, aufgetrennt. Der Brüdenstrom BS2 wird mit dem Verdichter V2 auf einen höheren Druck gebracht und anschließend als Strom VB2 zur Energieübertragung auf den Strom im Sumpfverdampfer SV2 geleitet. Der Strom VB2 wird anschließend zu einem Flashbehälter gefahren, wo durch die Entspannung eine flüssige Phase FP2 anfällt. Diese flüssige Phase (FP2) wird in die beiden Ströme FP2b, der als Rücklauf zur zweiten Destillationskolonne DK2 zurückgeführt wird, und einen Isobutan-Strom (4), der aus dem Verfahren ausgeschleust wird, aufgetrennt. Enthält der Feed der DK1 Spuren von Wasser, so kann dieses als schwere wässrige Phase (5) aus den Flash-Behältern der DK1 und der DK2 abgetrennt werden. Damit kann der Wassergehalt der Produktströme 2 und 3 minimiert werden.

Fig. 5 zeigt eine weitere Ausführungsform gemäß der vorliegenden Erfindung. Der Unterschied zur Fig. 4 besteht darin, dass der Strom FP1a nicht direkt zur Destillationskolonne DK2 geführt wird. Stattdessen enthält der Storm noch gewissen Mengen an Isobuten, die bei der Abtrennung in der DK2 im Produktstrom (3) anfallen und diesen verunreinigen würden. Deshalb wird der Strom FP1a zu einer MTBE- oder ETBE-Einheit gefahren, wo zunächst eine Umsetzung des Isobutens mit Methanol zu MTBE oder Ethanol zu ETBE und anschließend eine Abtrennung von MTBE stattfindet, beispielsweise unter Beteiligung einer Reaktivdestillationskolonne. Der aus der MTBE-Einheit erhaltene Strom FP1c enthält so gut wie kein MTBE und wird, wie für Fig. 4 beschrieben, in der Destillationskolonne DK2 aufgetrennt.

Fig. 6 zeigt eine weitere Ausführungsform gemäß der vorliegenden Erfindung. Der Unterschied zur Fig. 4 besteht einerseits darin, dass zwei zusätzliche Wärmetauscher (WT1, WT2) vorhanden sind, über die Energie von den Strömen VB1 (in WT1) und VB2 (in WT2) zur Überhitzung auf die Ströme BS1 (in WT1) und BS2 (in WT2) übertragen wird. Hierdurch kann verhindert werden, dass die Brüdenströme bei der Verdichtung ins Zweiphasengebiet gehen und sichergestellt werden, dass sich keine Tropfen bilden, die den Verdichter beschädigen können. Außerdem können die Ströme VB1 und VB2 in den Wärmeübertragern VW1 und VW2 zusätzlich Energie an die Feedströme der DK1 und DK2 abgeben, wodurch der Energiebedarf der Sumpfheizer SV1 und SV2 und damit die Antriebsleistung der Verdichter V1 und V2 verringert wird. Ein weiterer Unterschied zur Fig. 4 besteht darin, dass auf den beiden Flashbehältern jeweils ein Kondensator (KO1, KO2) zur Regelung des Drucks installiert ist. Zusätzlich können die Kondensatoren auch für den Anfahrprozess verwendet werden.

Fig. 7 zeigt eine weitere Ausführungsform der vorliegenden Erfindung. Der Unterschied zur Fig. 6 besteht darin, dass der Strom FP1a nicht direkt zur Destillationskolonne DK2 geführt wird. Stattdessen enthält der Storm noch gewissen Mengen an Isobuten, die bei der Abtrennung in der DK2 im Produktstrom (3) anfallen und diesen verunreinigen würden. Deshalb wird der Strom FP1a zu einer MTBE- oder ETBE-Einheit gefahren, wo zunächst eine Umsetzung des Isobutens mit Methanol zu MTBE oder Ethanol zu ETBE und anschließend eine Abtrennung von MTBE stattfindet, beispielsweise unter Beteiligung einer Reaktivdestillationskolonne. Der aus der MTBE-Einheit erhaltene Strom FP1c enthält so gut wie kein MTBE wird, wie oben beschrieben, in der Destillationskolonne DK2 aufgetrennt.

Fig. 8 zeigt eine weitere Ausführungsform der vorliegenden Erfindung. Der Unterschied zur Fig. 4 besteht darin, dass hier ein Verdichter (V3) mit mehreren Verdichtermaschinen eingesetzt wird. So ist es möglich, dass die Verdichtung von BS1 und BS2 unabhängig voneinander in einem Verdichter stattfindet. Die Abtrennung der schweren wässrigen Phase (5) erfolgt hier zudem über einen Euter, was über die Ausbuchtung angedeutet ist.

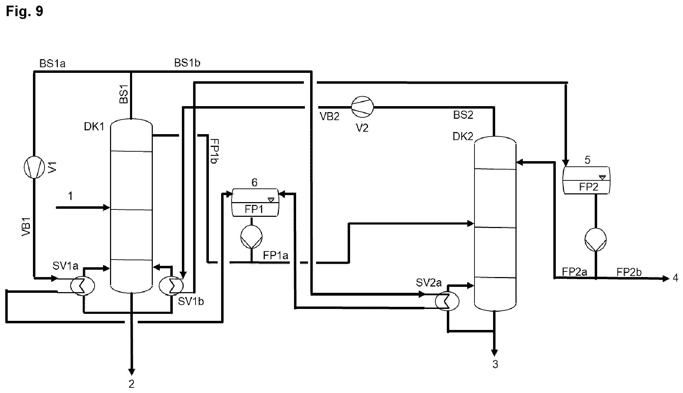

Fig. 9 zeigt eine alternative Ausgestaltung, bei der die beiden Destillationskolonnen unterschiedliche Drücke aufweisen und die Brüdenverdichtung anders verknüpft ist. Der Raffinat-2-Strom (1) wird zur ersten Destillationskolonne DK1 geführt und dort in einen Brüdenstrom BS1, der zumindest 1-Buten und Isobutan umfasst und am Kopf der DK1 entnommen wird, und in einen Sumpfstrom (2), der zumindest 1-Buten und 2-Buten umfasst und am Sumpf der DK1 entnommen wird. Der Brüdenstrom BS1 wird in die beiden Brüdenströme BS1a und BS1b aufgetrennt. BS1a wird mit dem Verdichter V1 auf einen höheren Druck gebracht und anschließend als Strom VB1 zur Energieübertragung auf den Strom im Sumpfverdampfer SV1a geleitet. Der zweite Teil BS1b des Brüdenstroms wird ohne zusätzliche Verdichtung zum Sumpfverdampfer SV2a geführt und überträgt dort Energie auf den dort vorhandenen Strom, der aus dem Sumpf kommend erhitzt und dann wieder zurückgeführt wird. Die beiden Ströme VB1 und BS1b werden nach der Energieübertragung zu einem Flashbehälter gefahren werden, wo durch die Entspannung eine flüssige Phase FP1 anfällt. Diese flüssige Phase wird in die beiden Ströme FP1a, der zur zweiten Destillationskolonne DK2 geführt wird, und FP1b, der als Rücklauf zur ersten Destillationskolonne geleitet wird, aufgetrennt. In der Destillationskolonne 2 werden die Ströme in einen Brüdenstrom BS2, der zumindest Isobutan umfasst und am Kopf der DK2 entnommen wird, und in einen Produktstrom (3), der zumindest 1-Buten umfasst und am Sumpf der DK2 entnommen wird, aufgetrennt. Der Brüdenstrom BS2 wird mit dem Verdichter V2 auf einen höheren Druck gebracht und anschließend als Strom VB2 zur Energieübertragung auf den Strom im Sumpfverdampfer SV1b geleitet. VB2 landet danach in einem Flashbehälter und kondensiert zumindest teilweise als flüssige Phase FP2, von der ein Teil FP2a aus Isobutan-Strom (4) das Verfahren verlässt und ein anderer Teil FP2b als Rücklauf zur zweiten Destillationskolonne zurückgeführt wird

### Beispiele

Für alle nachfolgenden Beispiele wurde ein Raffinat 2-Strom von 55t/h eingesetzt. Der Raffinat-2-Stroms hat die folgende Zusammensetzung: 1-Buten 45,1 % / n-Butan 22,4% / trans-2-Buten 15,9% / cis-2-Buten 8,9% / iso-Butan 7,4% / iso Buten 300 ppm und Wasser 590 ppm.

Die für den Betrieb, der in den Beispielen dargestellten Anlagen zur Abtrennung von 1-Buten aus Raffinat 2 notwendige Menge an Energie wurde anhand einer Simulation mittels Aspen V10 errechnet. Die Stoffdaten wurden durch Betriebsdaten und Betriebsversuche validiert.

### Beispiel 1 (nicht erfindungsgemäß

In der in Fig. 1 dargestellten Ausführungsform wird das Raffinat 2 in der oben angegebenen Zusammensetzung zur Destillationskolonne DK1 gefahren. Die Kolonne wird bei einem Kopfdruck von 6 bar abs. betrieben. Die Kopftemperatur beträgt 43°C. Es werden 140 theoretische Böden berücksichtigt. Über den Sumpf der Kolonne wird ein an 1-Buten abgereicherter Strom (Raffinat 3) abgezogen. Die Sumpftemperatur beträgt 62°C. Es wird ein Druckverlust von 1000 mbar in der DK1 berücksichtigt. Über den Kopf wird ein Destillatstrom in einer Menge von 15,2 t/h abgezogen, der etwa 77 wt% 1-Buten und etwa 22 wt% iso-Butan enthält. Für die Destillation in der DK1 ist eine Heizleistung von 15 MW notwendig, die über den Sumpfverdampfer SV1 mittels Heizdampf in die Kolonne eingetragen wird.

Die zweite Destillationskolonne DK2 wird in ähnlicher Weise betrieben. Zur Trennung des 1-Butens vom Isobutan aus dem Destillatstrom der DK1 wird in der zweiten Kolonne DK2 eine Heizleistung von 10,2 MW durch Heizdampf im Sumpfverdampfer SV2 eingebracht. Im Sumpf der DK2 werden 11,6 t/h Produktstrom abgezogen mit einer Reinheit von 99,6 wt% 1-Buten. Im Kopf der DK2 werden 3,6 t/h abgezogen. Der Destillatstrom besteht zu 95% aus iso-Butan. Die Kolonne wird ebenfalls bei einem Kopfdruck von 6 bar abs und einer Kopftemperatur von 40°C betrieben. Der Druckverlust beträgt bei 140 theoretischen Böden 1000 mbar. Es stellt sich eine Sumpftemperatur von 56,6°C ein.

Insgesamt müssen in der in Fig. 1 dargestellten Verschaltung 25,2 MW extern bereitzustellender Heizdampf eingesetzt werden.

### Beispiel 2 (nicht erfindungsgemäß)

Ein weiterer Versuch wurde mit der in Fig. 2 gezeigten Verschaltung durchgeführt. Im Unterschied zur

Fig. 1 und dem Beispiel1 ist eine Brüdenverdichtung an der Destillationskolonne DK1. Hierzu wird der Brüdenstrom der DK1 BS1 mittels dem Verdichter V1 auf 11 bar abs. verdichtet. Es entsteht der verdichtete Brüdenstrom VB1, der an dem Sumpfverdampfer SV1 kondensieren kann. Bei 11 bar abs. kondensiert der verdichtete Brüdenstrom bei 73°C. Für den Vorgang des Verdichtens wird eine elektrische Leistung von 1,6 MW benötigt. Über den SV1 können 15 MW übertragen werden.

Insgesamt müssen in der in Fig. 2 dargestellten Verschaltung 10,2 MW extern bereitzustellender Heizdampf und 1,6 MW elektrische Leistung eingesetzt werden.

### Beispiel 3 (nicht erfindungsgemäß)

Ein weiterer Versuch wurde mit der in Fig. 3 gezeigten Verschaltung durchgeführt. Im Unterschied zur Fig. 1 und dem Beispiel 1 erfolgt eine Brüdenverdichtung an der Destillationskolonne DK2. Hierzu ist eine elektrische Leistung von 1,0 MW notwendig, um den Brüden BS2 auf ein höheres Druckniveau von 11,7 bar abs. zu verdichten und um die Kondensationswärme so am SV2 nutzbar zu machen. Es können 10,2 MW übertragen werden.

Insgesamt müssen in der in Fig. 3 dargestellten Verschaltung 15 MW extern bereitzustellender Heizdampf und 1,0 MW elektrische Leistung eingesetzt werden,

### Beispiel 4 (erfindungsgemäß)

Ein weiterer Versuch wurde mit der in Fig. 4 gezeigten Verschaltung durchgeführt. Hier ist die erfindungsgemäße Wärmeintegration für beide Destillationskolonnen DK1 und DK2 vorgesehen. Hierzu kann sowohl ein einziger Verdichter mit mindestens zwei Stufen installiert oder zwei individuelle Verdichter eingesetzt werden. Beide Brüdenströme werden auf die geforderten Druckniveaus (s. Beispiele 2 und 3) verdichtet, um eine treibende Temperaturdifferenz ca. 10 K zu erzeugen und die Kondensationswärme so in den Prozess zu rezirkulieren. So können beide Kolonnen DK1 und DK2 in der gezeigten Verschaltung vollständig elektrifiziert werden.

In der in Fig. 4 dargestellten Ausführungsform muss kein extern bereitzustellender Heizdampf eingesetzt werden. Es werden lediglich 2,6 MW elektrische Leistung zum Betrieb der Verdichter benötigt.

### Beispiel 5 (erfindungsgemäß)

Ein weiterer Versuch wurde mit der in Fig. 6 gezeigten Verschaltung durchgeführt, bei der u. a. im Unterschied zur Fig. 4 zusätzlich die Brüdenströme BS1 und BS2 und die Feedströme vorgewärmt werden. Exzesswärme, die durch den Eintrag der elektrischen Leistung entsteht, muss nicht über eine zusätzliche Kühlung entzogen werden, sondern wird zur Vorwärmung der Brüdenströme in den Wärmetauschern WT1 und WT2 und der Feedströme in den Vorwärmern VW1 und VW2 verwendet. Demzufolge kann die in den Verdichtern benötigte elektrische Leistung von 2,6 MW im Beispiel 4 auf insgesamt 2,3 MW reduziert werden. Extern bereitzustellender Heizdampf muss hier ebenfalls nicht eingesetzt werden.

### Beispiel 6 (erfindungsgemäß)

Ein weiterer Versuch wurde mit der in Fig. 7 gezeigten Verschaltung durchgeführt, bei der im Unterschied zur Fig. 6 eine MTBE-Einheit vorhanden ist. In den hier aufgeführten Verschaltungen geht das Isobuten über Kopf der DK1 und bleibt im Sumpf der DK2. Es folgt also dem 1-Buten und ist in dieser Verschaltung kaum vom 1-Buten zu trennen. Demzufolge findet eine Aufkonzentration statt. Wird nun eine zweite MTBE-Stufe zwischen der DK1 und der DK2 berücksichtigt, so kann die Isobutenkonzentration im 1-Buten reduziert werden. Die Abtrennung von n-Butan in der Kolonne DK1 muss deshalb nicht mehr so scharf gefahren werden. Außerdem kann mehr Iso-Butan im Sumpf belassen werden. Insgesamt können so weitere 200 kW elektrische Leistung eingespart werden.

### Beispiel 7 (nicht erfindungsgemäß)

In dieser Ausführungsform wird eine direkte Wärmeintegration zwischen der Kolonne DK1 und DK2 durchgeführt (wie in DE 10 2005 062 700 A1 offenbart) und erfindungsgemäß um eine mehrstufige Brüdenverdichtung ergänzt (s. Fig. 9). Hierbei wird die Kolonne DK1 bei einem Kopfdruck von 11 bar und die Kolonne DK2 von 7 bar betrieben. So kann mit einem Teil des Brüdenstroms BS1 über den Sumpfverdampfer SV2a an der DK2 Wärme übertragen werden. Eine Kondensationswärme von 4,0 MW der DK1 wird über eine einstufige Brüdenverdichtung mittels Verdichter V1 nutzbar gemacht und über einen zweiten Sumpfverdampfer SV1a an der DK1 eingetragen. Dafür müssen 382 kW elektrische Leistung an dem Verdichter V1 aufgebracht werden. Weiterhin wird der Brüden der DK2 verdichtet, um die Kondensationswärme von 9,9 MW der DK2 für die DK1 nutzbar zu machen. Die über den Verdichter V2 aufgewertete Kondensationswärme wird über einen weiteren Sumpfverdampfer SV1b übertragen. Dafür ist eine elektrische Leistung von 2,6 MW erforderlich. Insgesamt können 11,5 MW über den SV1b durch Einsatz von 2,98 MW elektrischer Energie übertragen werden.

Nachfolgend sie die Ergebnisse der Beispiele 1 bis 7 in der Tabelle 1 zusammengefasst.

**Tabelle 1: Zusammenfassung der Beispiele**

| | Bsp. 1 | Bsp.2 | Bsp. 3 | Bsp. 4 | Bsp. 5 | Bsp. 6 | Bsp. 7 |
|---|---|---|---|---|---|---|---|
| Extern bereitzustellende Heizleistung [MW] | 25,2 | 10,2 | 15 | 0 | 0 | 0 | 0 |
| Elektrische Leistung Verdichter [MW] | 0 | 1,6 | 1,0 | 2,6 | 2,3 | 2,1 | 2,98 |

Es zeigt sich, dass die erfindungsgemäße Ausgestaltung des Verfahrens der 1-Butenabtrennung dazu führt, dass deutlich weniger externe Heizleistung eingesetzt werden muss als bei der bekannten Lösung. Das Einsparpotential ist also erheblich. Die dazu zusätzlich erforderliche elektrische Leistung zum Betrieb der Verdichter ist deutlich geringer und kann bei Einsatz von grünem Strom einen CO₂-neutralen Betrieb ermöglichen.

Es zeigt sich außerdem, dass eine Ausführungsform, bei der die beiden Destillationskolonnen DK1 und DK2 bei unterschiedlichem Druck gefahren werden, nachteilig ist, weil es den Einsatz einer höheren Menge an elektrischer Energie erfordert.

## Patentansprüche

1. Verfahren zur Abtrennung von 1-Buten aus einem Raffinat-2-Strom, der zumindest 1-Buten, 2-Buten, n-Butan, Isobuten und Isobutan enthält, in einer Abtrenneinheit, die mindestens zwei Destillationskolonnen DK1 und DK2 umfasst, wobei
die erste Destillationskolonne DK1 mindestens einen Sumpfverdampfer SV1 und die zweite Destillationskolonne DK2 mindestens einen Sumpfverdampfer SV2 aufweist;
der Sumpfverdampfer SV1 mit einem Strom gespeist wird, der am unteren Ende der DK1 entnommen wird und nach Durchlaufen des jeweiligen Sumpfverdampfers wieder zur DK1 geführt wird;
der Sumpfverdampfer SV2 mit einem Strom gespeist wird, der am unteren Ende der DK2 entnommen wird und nach Durchlaufen des jeweiligen Sumpfverdampfers wieder zur DK2 geführt wird; wobei das Verfahren die folgenden Schritte umfasst
(a) der Raffinat-2-Strom zur ersten Destillationskolonne DK1 geleitet wird und in der DK1 in mindestens einen Brüdenstrom BS1, der zumindest 1-Buten und Isobutan umfasst und am Kopf der DK1 entnommen wird, und in mindestens einen Sumpfstrom, der zumindest 1-Buten, n-Butan und 2-Buten umfasst und am Sumpf der DK1 entnommen wird, aufgetrennt wird;
(b) zumindest ein Teil des Brüdenstroms BS1 verdichtet wird, wodurch ein gegenüber dem Brüdenstrom BS1 verdichteter Strom VB1 entsteht;
(c) Energie vom verdichteten Strom VB1 auf den Strom im Sumpfverdampfer SV1 übertragen wird;
(d) der Strom VB1 zumindest teilweise zur zweiten Destillationskolonne DK2 gleitet werden und in der DK2 in mindestens einen Brüdenstrom BS2, der zumindest Isobutan umfasst und am Kopf der DK2 entnommen wird, und in mindestens einen Sumpfstrom, der zumindest 1-Buten umfasst und am Sumpf der DK2 entnommen wird, aufgetrennt wird;
(e) der Brüdenstrom BS2 zumindest teilweise verdichtet wird, wodurch ein gegenüber dem Brüdenstrom BS2 verdichteter Strom VB2 entsteht; und
(f) Energie vom verdichteten Strom VB2 auf den Strom im Sumpfverdampfer SV2 übertragen wird.

2. Verfahren nach Anspruch 1, wobei die Destillationskolonnen DK1 und DK2 bei gleichem Kopfdruck oder einem ähnlichen Kopfdruck betrieben werden, wobei sich die Kopfdrücke im Falle ähnlicher Kopfdrücke um maximal 20%, vorzugsweise maximal 15% unterscheiden.

3. Verfahren nach Anspruch 1 oder 2, wobei zwischen den Destillationskolonnen DK1 und DK2 eine MTBE- oder ETBE-Synthese angeordnet ist, die mit dem Strom VB1 gespeist wird.

4. Verfahren nach einem der Ansprüche 1 bis 3, wobei der Strom VB1 einem Flashbehälter geführt und dort entspannt werden, wodurch eine flüssige Phase FP1 des Stroms VB1 anfällt.

5. Verfahren nach Anspruch 4, wobei zumindest ein Teil FP1a der flüssigen Phase FP1 zur Destillationskolonne DK2 geleitet wird, vorzugsweise mittels einer Pumpe.

6. Verfahren nach Anspruch 5, wobei ein anderer Teil FP1b der flüssigen Phase FP1 als Rücklauf zur Destillationskolonne DK1 zurückgeführt wird.

7. Verfahren nach einem der vorhergehenden Ansprüche, wobei Energie vom Strom FP1b auf den Raffinat-2-Strom übertragen wird, bevor der Raffinat-2-Strom in die erste Destillationskolonne DK1 eingeleitet wird.

8. Verfahren nach einem der vorhergehenden Ansprüche, wobei im Sumpf der ersten Destillationskolonne DK1 eine Temperatur im Bereich von 40 bis 110 °C, vorzugsweise 50 bis 100 °C vorliegt.

9. Verfahren nach einem der vorhergehenden Ansprüche, wobei im Sumpf der zweiten Destillationskolonne DK2 eine Temperatur im Bereich von 30 bis 100 °C, vorzugsweise 45 bis 80 °C vorliegt.

10. Verfahren nach einem der vorhergehenden Ansprüche, wobei die Destillationskolonne DK1 zwischen 150 und 300 Böden aufweist.

11. Verfahren nach einem der vorhergehenden Ansprüche, wobei die Destillationskolonne DK2 zwischen 150 und 300 Böden aufweist.

12. Verfahren einem der vorhergehenden Ansprüche, wobei für die Verdichtung der Ströme BS1 und BS2 nur ein einziger Verdichter eingesetzt wird.

13. Verfahren nach einem der vorhergehenden Ansprüche, wobei der Strom VB2 in einen Flashbehälter entspannt wird, wo eine flüssige Phase FP2 anfällt.

14. Verfahren nach Anspruch 12, wobei zumindest ein Teil FP2a der flüssigen Phase FP2 als Produktstrom aus dem Verfahren ausgeschleust wird, vorzugsweise mittels einer Pumpe.

15. Verfahren nach Anspruch 13, wobei ein anderer Teil FP2b der flüssigen Phase FP2 als Rücklauf zur Destillationskolonne DK2 zurückgeführt wird.
